# EUROPÄISCHE PATENTSCHRIFT

(11) **EP 0 646 562 B1**
(45) Veröffentlichungstag und Bekanntmachung des Hinweises auf die Patenterteilung: **27.08.1997**
(21) Anmeldenummer: 94114851.2
(22) Anmeldetag: 21.09.1994
(51) Int. Cl.: C07C 29/17, B01J 23/60, B01J 23/62, B01J 37/02

(54) **Verfahren zur selektiven Hydrierung von Butindiol-1,4 zu Buten-2-diol-1,4 und dafür geeigneter Katalysator**
Hydrogenation of 2-butyne-1,4-diol to 2-butene-1,4-diol and suitable catalyst
Hydrogénation de 2-butyne-1,4-diol en 2-butène-1,4-diol et catalyseur pour cette hydrogénation

(30) Priorität: 30.09.1993 DE 4333293
(43) Veröffentlichungstag der Anmeldung: 05.04.1995
(73) Patentinhaber: BASF Aktiengesellschaft, 67063 Ludwigshafen (DE)
(72) Erfinder: Bröcker, Franz Josef, Dr., D-67061 Ludwigshafen (DE); Becker, Rainer, Dr., D-67098 Bad Dürkheim (DE); Menger, Volkmar, Dr., D-67434 Neustadt (DE); Stops, Peter, D-67122 Altrip (DE)

(56) Entgegenhaltungen:
- EP-A- 0 412 415
- DE-A- 2 818 260
- DE-A- 3 114 240

## Beschreibung

Die Erfindung betrifft ein Verfahren zur Hydrierung von Butindiol zu Butendiol unter Verwendung eines Drahtnetzkatalysators, auf den nacheinander Palladium und Blei oder Cadmium aufgebracht worden sind.

Die Hydrierung von Butindiol zu Butendiol ist Gegenstand eines umfangreichen Standes der Technik.

So wird in GB-A 871 804 eine verbesserte selektive Hydrierung von Acetylenverbindungen in Suspensionsfahrweise mit einem Pd-Katalysator, der mit Salzlösungen der Metalle Zn, Cd, Hg, Ga, In oder Tl behandelt wurde, beschrieben.

Ferner wird in der DE-A 24 31 929 ein Verfahren zur Herstellung von Buten-2-diol-1,4 durch Hydrieren von Butindiol in wäßriger Lösung an einem Katalysator, der Pd und eines der Elemente Zn oder Cd und wenigstens eines der Elemente Bi oder Te enthält, beschrieben. Als Katalysatorträger wird Bimsstein oder Aluminiumoxid verwendet.

Für die selektive Hydrierung der Dreifachbindung in Vitamin- und Riechstoffvorprodukten werden üblicherweise und wie z.B. in US-A 2 681 938 beschrieben, Blei-dotierte Pd-Katalysatoren, sogenannte Lindlar-Katalysatoren eingesetzt. Vielfach werden diese noch mittels Schwefelverbindungen desaktiviert, um die Selektivität zu steigern (JP-A 120 657/81). Aus DE-A 26 19 660 ist schließlich ein Verfahren zur Herstellung von Butendiol bekannt, bei dem Butindiol in einem inerten Lösungsmittel in Anwesenheit eines Katalysators, der mit Kohlenmonoxid behandeltes metallisches Pd enthält, hydriert wird.

Auch die Verwendung eines Pd/BaSO₄-Katalysators zur Herstellung von Butendiol ist aus der DE-A 26 05 241 bekannt.

Alle genannten Verfahren zur Herstellung von Butendiol durch selektive Hydrierung von Butindiol haben den Nachteil, daß ein suspendierter Katalysator Verwendung findet und der Katalysator nach erfolgter Hydrierung durch Absetzen und Filtrieren vom Reaktionsprodukt abgetrennt werden muß.

Es hat sich nun gezeigt, daß die vollständige Abtrennung des pulverförmigen Katalysators nur mit sehr großem Aufwand möglich ist. Spuren von Katalysatorresten im Butendiol verursachen aber bei der Weiterverarbeitung erhebliche Schwierigkeiten, so daß es nicht an Versuchen gefehlt hat, einen Festbettkatalysator für die Hydrierung der Dreifachbindung im Butindiol zu entwickeln.

Aus der EP-A 04 12 415 ist ein solcher Festbettkatalysator für die Hydrierung von Hydro-Dehydrolinalool zu Hydro-Linalool bekannt, der als Aktivkomponente Palladium und als Inhibitor Bismut enthält.

Ein nach den Angaben dieser EP-A 04 12 415 hergestellter Pd/Bi-Katalysator liefert aber bei der Hydrierung von Butindiol zu Butendiol bei vollem Umsatz ein Hydrierprodukt mit einem zu hohen Rückstand von 6,75 % (s. Vergleichsbeispiel), vermutlich durch Polymerenbildung als Folgereaktion.

DE-A 31 14 240 betrifft ein Verfahren zur Herstellung von Ethylenalkoholen mit 4 bis 10 Kohlenstoffatomen durch die Hydrierung der entsprechenden Propargylalkohole in flüssiger Phase an einem Membrankatalysator aus einer Legierung, die aus Palladium und Ruthenium bei einem Masseverhältnis derselben von 90 bis 94 zu 10 bis 6 besteht. Derartige Membrankatalysatoren sind infolge der geringen Folienstärke verschleißanfällig und aufgrund ihrer Funktionsweise, Diffusion des Wasserstoffs durch die Membran zum Edukt, ist die mit ihnen erzielbare Raum-Zeit-Ausbeute gering.

Es bestand nun die Aufgabe, für die selektive Hydrierung von Butindiol zu Butendiol einen Festbettkatalysator zu entwickeln, der die Nachteile der Hydrierung mit einem suspendierten Katalysator vermeidet und der ein Minimum an Nebenprodukten liefert.

Es wurde nun gefunden, daß sich die Rückstandsbildung beim Verfahren zur Herstellung von Buten-2-diol-1,4 durch Hydrierung von Butin-2-diol-1,4 in wäßriger Lösung an einem Palladium enthaltenden Festbettkatalysator, der mit Blei oder Cadmium dotiert ist, minimieren läßt, wenn man einen Katalysator verwendet, bei dem Pd und Pb bzw. Pd und Cd nacheinander durch Aufdampfen oder Sputtern auf einem Metalldrahtnetz oder einer Metallfolie als Träger aufgebracht worden sind und anschließend 0,5 bis 2 Stunden eine Formierung des Katalysators bei 200 bis 800°C an der Luft durchgeführt worden ist und der vor dem Gebrauch mit Wasserstoff bei Temperaturen von 20 bis 250°C reduziert worden ist. Diese so hergestellten Katalysatoren zeigen im Vergleich zu Bismut-dotierten Palladium-Katalysatoren eine erhöhte Aktivität und Selektivität. Die erhöhte Aktivität ermöglicht eine Hydrierung bei Temperaturen zwischen 20 und 100°C, vorzugsweise 40 und 80°C. Da die Bildung von trans-Butendiol mit steigender Temperatur zunimmt und ein hoher cis-Anteil im Butendiol erwünscht ist, ist eine Hydrierung bei möglichst tiefer Temperatur vorteilhaft.

Die erfindungsgemäßen Katalysatoren werden durch Aufdampfen bzw. Aufsputtern der Aktivkomponente Pd und der Inhibitoren Cd bzw. Pb auf einen folien- bzw. gewebeartigen Metallträger hergestellt.

Besonders bewährt haben sich metallische Folien bzw. Gewebe aus Werkstoffen mit den Werkstoffnummern: 1.4767, 1.4401 und 1.4301. Die Bezeichnung dieser Werkstoffe mit den obengenannten Werkstoffnummern folgt den Angaben der Werkstoffnummern in der "Stahleisenliste", herausgegeben vom Verein Deutscher Eisenhüttenleute; 8. Aufl., S. 87, S. 89 und S. 106, Verlag Stahleisen mbH, Düsseldorf 1990. Der Werkstoff mit der Werkstoffnummern 1.4767 ist auch unter der Bezeichnung Kanthal bekannt.

Diese metallischen Trägermaterialien werden durch oxidative Temperung, vorzugsweise an der Luft, bei Temperaturen von 600 bis 1100°C, vorzugsweise 700 bis 1000°C vorbehandelt und anschließend mit der Aktivkomponente und dem Inhibitor beschichtet. Nach der Beschichtung wird eine thermische Formierung an der Luft durchgeführt und so das eigentliche Katalysatormaterial erhalten. Zu dieser Formierung wird das beschichtete Trägermaterial bei Temperaturen von 200 bis 800, vorzugsweise 300 bis 700°C 0,5 bis 2 h an der Luft erhitzt. Das so gefertigte Katalysatormaterial wird anschließend durch Verformung zu Monolithen verarbeitet. Nach der Reduktion des Katalysators mit Wasserstoff bei Temperaturen von 50 bis 250, vorzugsweise 80 bis 150°C, die man vorteilhaft im Reaktor durchführt, ist der Katalysator für die Hydrierung von Butindiol einsatzbereit.

Erfindungsgemäß werden Katalysatoren verwendet, die durch Aufdampfen oder Aufsputtern von zunächst Palladium und daran anschließend von Blei oder Cadmium auf das Trägermaterial hergestellt worden sind. Dazu wird zunächst das Palladium in einer Menge von im allgemeinen 20 bis 300 mg/m², vorzugsweise von 40 bis 200 mg/m², und danach Blei in einer Menge von im allgemeinen 10 bis 60 mg/m², vorzugsweise von 20 bis 40 mg/m², oder Cadmium in einer Menge von 10 bis 50 mg/m², vorzugsweise von 15 bis 30 mg/m², auf das Trägermaterial aufgedampft oder aufgesputtert.

Die Methoden des Aufdampfens und Aufsputterns von Metallen im Vakuum sind im einzelnen in "Handbook of Thin Film Technology", Maissel und Glang, McGraw Hill, New York, 1970, "Thin Film Processes", J.L. Vossen u. W. Kern, Academic Press N.Y. sowie in EP-A 0 198 435 beschrieben, worauf hiermit Bezug genommen wird.

Die Hydrierung wird zweckmäßig mit einer z.B. 50 %igen wäßrigen Butindiollösung in einer Druckapparatur in Rieselfahrweise mit einer konstanten Querschnittsbelastung von 20 bis 120, insbesondere z.B. von 60 m³/m² x h durchgeführt. Diese Querschnittsbelastung garantiert eine gleichmäßige Benetzung des Katalysators.

Die folgenden Beispiele zeigen die Hydrierergebnisse mit unterschiedlichen Trägermaterialien im Vergleich zu dem vorbekannten Palladium/Bismut-Katalysator.

In den folgenden Beispielen sind alle Mengenprozentangaben Gewichtsprozent, alle Ausbeuten Prozent der Theorie.

### Beispiel 1

Ein Drahtgewebe in Leinenbindung aus dem Werkstoff mit der Nr. 1.4767 mit einer Maschenweite von 0,18 mm und einem Drahtdurchmesser von 0,112 mm wurde 5 h bei 1000°C an der Luft getempert. Anschließend wurde das so vorbehandelte Trägergewebe in einer Elektronenstrahlbedampfungsanlage nacheinander mit 92 mg Palladium/m² (Gewebefläche) und 21,3 mg Blei/m² beschichtet. Das beschichtete Gewebe wurde in einer Muffel 0,5 Stunden an der Luft bei 600°C zur Formierung des Katalysators erhitzt. Aus dem so hergestellten Katalysatorgewebe wird ein monolithischer Körper geformt. Dazu wurde mittels einer Zahnradwalze ein Teil des Gewebes gewellt. Dieses gewellte Gewebe wurde mit einem glatten Gewebestreifen zusammengelegt und aufgewickelt. Man erhielt so einen monolithischen Formkörper, der durch Punktschweißen gefestigt wurde. In der Hydrierapparatur wurde dieser Katalysator bei 150°C drucklos eine Stunde mit Wasserstoff reduziert und dann nach Abkühlung zur Hydrierung einer 50 %igen wäßrigen Butindiollösung bei einem H₂-Partialdruck von 15 bar und einer Hydriertemperatur von 60°C verwendet. Bei einer Querschnittsbelastung von 60 [m³/m² x h] wurde die 50 %ige wäßrige Lösung in Rieselfahrweise hydriert. Die Raumzeitausbeute betrug 1,0 [1 : 1 Kat. x h] bezogen auf die 50 %ige Lösung und bezogen auf die Katalysatorgewebefläche 0,42 [1 : m² x h]. Das Hydrierprodukt (organischer Anteil ohne H₂O) enthielt 98 % Butendiol mit einem trans-Butendiolanteil von 1,76 % und 1,8 % Rückstand.

### Beispiel 2

Ein Drahtgewebe aus dem Werkstoff mit der Werkstoff-Nr. 1.4767 wurde, wie in Beispiel 1 beschrieben, vorbehandelt und anschließend mit 92 mg Pd/m² und 16,2 mg Cd/m² bedampft und 0,5 Stunden bei 300°C getempert. Nach Verformung zum Monolithen wurde eine 50 %ige wäßrige Butindiollösung bei 55CC und 15 bar H₂-Partialdruck, wie in Beispiel 1 beschrieben, hydriert. Die gemessene Raumzeitausbeute betrug auf die Lösung bezogen 1,57 [1 : 1 Kat. x h] bzw. 0,69 [1 : m² x h]. Für das organische Reaktionsprodukt ergab sich ein Butendiolgehalt von 97,1 % mit einem trans-Anteil von 1,12 % und eine Rückstandsmenge von 1,37 %.

### Beispiel 3

V2A-Gewebe, Werkstoff-Nr. 1.4401, mit einer Maschenweite von 200 µm und einem Drahtdurchmesser von 125 µm wurde 3 h bei 850°C getempert. Anschließend wurde das so vorbehandelte Gewebe wie in Beispiel 1 beschrieben mit 92 mg Pd/m² und 21,3 mg Pb/m² beschichtet und danach 0,5 Stunden bei 300°C nachgetempert. Wie in Beispiel 1 und 2 beschrieben, wurde das zu einem Monolithen verarbeitete Katalysatorgewebe für die Hydrierung von Butindiol eingesetzt. Die Raumzeitausbeute betrug, bezogen auf die wäßrige Lösung, 1,6 [1 : 1 Kat. x h] bzw. 0,6 [1 : m² x h] bezogen auf die Katalysatorgewebefläche. Das Hydrierprodukt enthielt 98 % Butendiol mit einem trans-Anteil von 1,7 % und einem Rückstand von 1,9 %.

### Beispiel 4

Wie in Beispiel 3 beschrieben, wurde ein Edelstahlgewebe mit der Werkstoff-Nr. 1.4301, einer Maschenweite von 125 µm und einer Drahtstärke von 100 µm 3 Stunden bei 800°C geglüht und danach mit 92 mg Palladium/m² (Gewebefläche) sowie 21,3 mg Blei/m² beschichtet. Das beschichtete Gewebe wurde zur Formierung der Luft in einer Stunde auf 300°C erhitzt und 30 Minuten auf dieser Temperatur gehalten. Nach Verformung zum Katalysatormonolithen wurde wie in Beispiel 1 bis 3 getestet. Bei einer Raumzeitausbeute von 1,7 [1 : 1 Kat. x h] bzw. 0,62 [1 : m² x h], lieferte die Hydrierung ein organisches Produkt, welches 98 % Butendiol mit einem trans-Anteil von 1,4 % und einem Rückstand von 2 % enthielt.

### Vergleichsbeispiel

Zum Vergleich wurde ein Palladium/Bismut-Katalysator nach EP-A 412 415 eingesetzt. Dazu wurde wie in Beispiel 1 beschrieben, das Drahtgewebe, Werkstoff-Nr. 1.4767 durch Tempern bei 1000°C vorbehandelt und anschließend mit 46 mg Pd/m² und 12,8 mg Bi/m² beschichtet. Das beschichtete Gewebe wurde 0,5 Stunden bei 600°C an der Luft getempert und dann zu einem Monolithen verformt. Die Hydrierung einer 50 %igen wäßrigen Butindiollösung wurde wie in Beispiel 1 bei einer Hydriertemperatur von 120°C durchgeführt. Die Raumzeitausbeute betrug 1,6 [1/1Kat. x h] bzw 0,7 [1/m² x h] bezogen auf die 50 %ige Lösung. Die Analyse des organischen Hydrierprodukts ergab einen Butendiolgehalt von 93 % und eine Rückstandsmenge von 6,75 %.

## Patentansprüche

1. Verfahren zur Herstellung von Buten-2-diol-1,4 durch Hydrierung von Butin-2-diol-1,4 in wäßriger Lösung an einem Palladium enthaltenden Festbettkatalysator, der mit Blei oder Cadmium dotiert ist, dadurch gekennzeichnet, daß man einen Katalysator verwendet, bei dem Pd und Pb bzw. Pd und Cd nacheinander durch Aufdampfen oder Sputtern auf einem Metalldrahtnetz oder einer Metallfolie als Träger aufgebracht worden sind und anschließend 0,5 bis 2 Stunden eine Formierung des Katalysators bei 200 bis 800°C an der Luft durchgeführt worden ist und der vor dem Gebrauch mit Wasserstoff bei Temperaturen von 20 und 250°C reduziert worden ist.

2. Verfahren nach Anspruch 1, dadurch gekennzeichnet, daß man metallische Träger mit den Werkstoffnummern 1.4767, 1.4401 und 1.4301 verwendet.

3. Verfahren nach den Anspruch 1, dadurch gekennzeichnet, daß man vor dem Aufbringen der aktiven Metalle die Träger an der Luft bei Temperaturen von 600 bis 1100°C, vorzugsweise 700 bis 1000°C glüht.

4. Verfahren nach Anspruch 1, dadurch gekennzeichnet, daß man auf das Trägermaterial Pd in einer Menge von 20 bis 300 mg/m², vorzugsweise 40 bis 200 mg/m² und danach Pb in einer Menge von 10 bis 60 mg/m², vorzugsweise 20 bis 40 mg/m² oder Cd in einer Menge von 10 bis 50 mg/m², vorzugsweise 15 bis 30 mg/m² aufdampft oder aufsputtert.

5. Verfahren nach den Anspruch 1, dadurch gekennzeichnet, daß man das beschichtete Katalysatormaterial an der Luft bei Temperaturen von 300 bis 700°C, 0,5 bis 2 Stunden formiert.

6. Verfahren nach Anspruch 1, dadurch gekennzeichnet, daß man den Katalysator vor dem Gebrauch mit Wasserstoff bei Temperaturen von 80 bis 150°C reduziert.

7. Katalysator in Form von Metallgeweben oder Metallfolien, hergestellt in dem zuerst Palladium und dann Blei oder Cadmium durch Aufdampfen oder Sputtern auf das Trägermaterial aufgebracht werden, dann das beschichtete Trägermaterial bei 200 bis 800°C für 0,5 bis 2 Stunden an der Luft erhitzt und anschließend bei 50 bis 250°C mit Wasserstoff reduziert wird.

8. Katalysator gemäß Anspruch 7 in Form von Metallgeweben oder Metallfolien aus den Werkstoffen mit den Nr. 1.4767, 1.4401 oder 1.4301, auf die nacheinander zuerst Palladium in Mengen von 20 bis 300 mg/m² Oberfläche und danach Blei in einer Menge von 10 bis 60 mg/m² und/oder Cadmium in einer Menge von 10 bis 50 mg/m² durch Aufdampfen oder Sputtern aufgebracht worden sind.

9. Katalysator gemäß Anspruch 7 dadurch gekennzeichnet, daß die Metallgewebe oder Metallfolien durch Prägen und Rollen zu Monolithen verformt sind.

## Revendications

1. Procédé de fabrication de butène-2-diol-1,4 par hydrogénation de butyne-2-diol-1,4 en solution aqueuse sur un catalyseur à lit fixe contenant du palladium, muni de plomb ou de cadmium, caractérisé en ce que l'on utilise un catalyseur pour lequel le Pd et le Pb, respectivement Pd et Cd, sont déposés, l'un après l'autre, sur un grillage métallique ou sur une feuille métallique servant de support, par dépôt en phase vapeur ou par pulvérisation, puis l'on effectue pendant 0,5 à 2 heures une formation du catalyseur à 200-800°C à l'air et on le réduit avant usage avec de l'hydrogène à des températures de 20 à 250°C.

2. Procédé selon la revendication 1, caractérisé en ce que l'on utilise des supports métalliques ayant les numéros de matériau 1.4767, 1.4401 et 1.4301.

3. Procédé selon la revendication 1, caractérisé en ce que l'on porte au rouge les supports, à l'air, à des températures de 600-1100°C, de préférence 700-1000°C, avant le dépôt des métaux actifs.

4. Procédé selon la revendication 1, caractérisé en ce que l'on dépose en phase vapeur ou par pulvérisation du Pd en une quantité de 20 à 300 mg/m², de préférence de 40 à 200 mg/m² et ensuite du Pb en une quantité de 10 à 60 mg/m², de préférence de 20 à 40 mg/m² ou du Cd en une quantité de 10 à 50 mg/m², de préférence 15 à 30 mg/m², sur le matériau du support.

5. Procédé selon la revendication 1, caractérisé en ce que l'on forme le matériau du catalyseur enduit, à l'air, à des températures de 300 à 700°C pendant 0,5 à 2 heures.

6. Procédé selon la revendication 1, caractérisé en ce que l'on réduit le catalyseur avant usage avec de l'hydrogène à des températures de 80 à 150°C.

7. Catalyseur sous forme de tissus métalliques ou de feuilles métalliques, fabriqué par dépôt sur le matériau du support de palladium d'abord, puis de plomb ou de cadmium par dépôt en phase vapeur ou par pulvérisation, puis le matériau du support enduit est chauffé à 200-800°C pendant 0,5 à 2 heures à l'air et ensuite réduit avec de l'hydrogène à 50-250°C.

8. Catalyseur selon la revendication 7 sous forme de tissus métalliques ou de feuilles métalliques à base de matériaux ayant les numéros 1.4767, 1.4401 et 1.4301, sur lesquels on dépose, en phase vapeur ou par pulvérisation, l'un après l'autre, du palladium en quantités de 20 à 300 mg/m² en surface, puis du plomb en une quantité de 10 à 60 mg/m² et/ou du cadmium en une quantité de 10 à 50 mg/m².

9. Catalyseur selon la revendication 7, caractérisé en ce que les tissus métalliques ou les feuilles métalliques sont transformées en monolithes par estampage et calandrage.

## Claims

1. A process for preparing butene-2-diol-1,4 by hydrogenating butyne-2-diol-1,4 in aqueous solution on a fixed-bed catalyst which contains palladium and is doped with lead or cadmium, wherein a catalyst is employed with which Pd and Pb or Pd and Cd have been applied successively by vapour deposition or sputtering onto a metal wire mesh or a metal foil as a support, followed by activation of the catalyst over a period from 0.5 to 2 hours at from 200 to 800°C in air, and which, prior to use, has been reduced with hydrogen at temperatures of 20 and 250°C.

2. A process as claimed in claim 1, wherein metallic supports of material numbers 1.4767, 1.4401 and 1.4301 are used.

3. A process as claimed in claim 1, wherein, prior to the active metals being applied, the supports are annealed in the air at temperatures of from 600 to 1100°C, preferably from 700 to 1000°C.

4. A process as claimed in claim 1, wherein Pd in an amount of from 20 to 300 mg/m², preferably from 40 to 200 mg/m², and then Pb in an amount of from 10 to 60 mg/m², preferably from 20 to 40 mg/m² or Cd in an amount of from 10 to 50 mg/m², preferably from 15 to 30 mg/m², are vapour-deposited or sputtered onto the support material.

5. A process as claimed in claim 1, wherein the coated catalyst material is activated in air at temperatures of from 300 to 700°C over a period from 0.5 to 2 hours.

6. A process as claimed in claim 1, wherein the catalyst, prior to use, is reduced with hydrogen at temperatures of from 80 to 150°C.

7. A catalyst in the form of metal gauzes or metal foils, prepared by first palladium and then lead or cadmium being applied to the support material by vapour deposition or sputtering, the coated support material then being heated at from 200 to 800°C over a period of from 0.5 to 2 hours in air and then being reduced with hydrogen at from 50 to 250°C.

8. A catalyst as claimed in claim 7 in the form of metal gauzes or metal foils made of the materials having No. 1.4767, 1.4401 or 1.4301, to which successively first palladium in amounts of from 20 to 300 mg/m² of surface area and then lead in an amount of from 10 to 60 mg/m² and/or cadmium in an amount of from 10 to 50 mg/m² have been applied by vapour deposition or sputtering.

9. A catalyst as claimed in claim 7, wherein the metal gauzes or metal foils have been formed into monoliths by stamping and rolling.
